# EUROPÄISCHE PATENTANMELDUNG

(11) **EP 1 839 651 A1**
(43) Veröffentlichungstag der Anmeldung: **03.10.2007**
(21) Anmeldenummer: 07103714.7
(22) Anmeldetag: 20.04.2000
(51) Int. Cl.: A61K 9/20

(54) **Verfahren zur Herstellung einer Wasserunlösslichen amorphen oder teilamorphen Retardmatrix**

(30) Priorität: 22.04.1999 DE 19918325
(62) Teilanmeldung aus: 00927010.9
(71) Anmelder: EURO-CELTIQUE S.A., 2330 Luxembourg (LU)
(72) Erfinder: Rein, Hubert, 53359, Rheibach/Flerzheim (DE); Steffens, Klaus-Jürgen, 53359, Rheibach/Flerzheim (DE)
(74) Vertreter: Glas, Holger

(57) **Zusammenfassung**

Die Erfindung betrifft ein Verfahren zur Herstellung von Arzneiformen oder Vorstufen davon mittels Extrusion, wobei die Arzneiform eine den Wirkstoffgehalt im wesentlichen enthaltende Matrix aufweist, die in ihren wesentlichen Eigenschaften durch die Extrusion ausgebildet wird, und ein Polysaccharid und/oder ein Derivat davon und/oder einen Komplex davon und/oder eine beliebige Mischung der vorgenannten Substanzen mit anderen Substanzen und/oder Sacchariden und/oder Derivaten davon als wesentlichen Bestandteil der Matrix sowie mindestens eine pharmazeutisch wirksame Substanz umfasst. Die Erfindung betrifft weiterhin eine Arznei form, welche eine den Wirkstoffgehalt im wesentlichen enthaltende Matrix, die in ihren wesentlichen Eigenschaften durch Extrusion ausgebildet ist, und ein Polysaccharid und/oder ein Derivat davon und/oder einen Komplex davon und/oder eine beliebige Mischung der vorgenannten Substanzen mit anderen Substanzen und/oder Sacchariden und/oder Derivaten davon als wesentlichen Bestandteil der Matrix sowie mindestens eine pharmazeutisch wirksame Substanz umfasst. Weiter betrifft die Erfindung die Verwendung der Arzneiform zur Herstellung von Granulaten für die Tablettierung und Kapselfüllung, zur Weiterverarbeitung mit Spritzgussverfahren, als Hilfsstoff bei der Direkttablettierung und/oder zur Herstellung von Monoblockarzneiformen.

## Beschreibung

Die Erfindung betrifft ein Verfahren zur Herstellung von Arzneiformen oder Vorstufen davon mittels Extrusion.

Die Erfindung betrifft weiterhin eine mittels Extrusionsverfahren herstellbare Arzneiform.

Die Extrusion ist insbesondere in der Klebstoffindustrie oder auch der Kunststoffverarbeitung-ein weit verbreiteter Prozeß zur Modifikation von Polysacchariden, insbesondere Stärken. Aus der Lebensmitteltechnologie ist die Extrusion zur Herstellung von stärkehaltigen Zubereitungen, wie etwa Nudeln, den sogenannten Erdnußflips oder diversen Süßigkeiten bekannt. Jedoch werden bei allen diesen Produkten die Herstellungsbedingungen so gewählt, daß aufgeschäumte, sogenannte gepoppte Produkte entstehen.

In der pharmazeutischen Technologie findet die Extrusion Verwendung zur Verarbeitung von Wachsen, Fettalkoholen, Fetten sowie verschiedenen Thermoplasten und Duroplasten. Beschrieben werden beispielsweise extrudierte Matrizes aus diversen Kunststoffverbindungen in der EP-A2-0 240 904, EP-A2-0 240 906 und in der EP-A2-0 358 105. Die Verarbeitung stärkehaltiger Mischungen zu pharmazeutischen Produkten (Kapselhüllen) mittels eines Spritzgußverfahrens beschreibt die EP-B2-0 118 240. Wenig bekannt ist in diesem Zusammenhang, wie die verwendeten Polysaccharide beim Extrudieren verändert werden.

Aus der EP-A-0 580 860 sind Extrusionsverfahren zur Herstellung von festen Dispersionen eines Wirkstoffs in einem Polymerträger bekannt. Unter den verwendbaren Rohstoffen sind generell auch Stärke und Stärkederivate erwähnt. Für Präparate mit Stärke als Hauptbestandteil eignet sich das dort beschriebene Verfahren jedoch nicht.

In der pharmazeutischen Technologie besitzen die Arzneiformen die oral aufgenommen werden, also beispielsweise Tabletten, Dragees oder Kapseln, mit Abstand die größte Bedeutung. Zu diesen zählen auch die sogenannten Retard-Arzneiformen, die eine relativ hohe Wirkstoffdosis enthalten und diese kontrolliert und gesteuert über einen längeren Zeitraum abgeben. Dies bedeutet für den Patienten, daß die Frequenz der Einnahme deutlich reduziert werden kann. Aus medizinisch-pharmakologischer Sicht liegt der Vorteil der Retard-Formen in einer sehr gleichmäßigen Wirkstoffkonzentration im Blut, die zu einer lang anhaltenden Wirkung und zu wenig Nebenwirkungen führt. Bei der Formulierung von Retard-Arzneiformen kommt der sogenannten Matrixform eine wichtige Bedeutung zu. Unter Matrix versteht man einen Formkörper, also beispielsweise eine Tablette oder ein Dragee, aus indifferenten Hilfsstoffen, aus dem im Magen-Darm-Trakt die Wirkstoffe kontrolliert abgegeben werden. Die Freisetzung der Wirkstoffe geschieht im allgemeinen teilweise über Diffusion, teilweise durch den langsamen Abbau der Matrix.

In der pharmazeutischen Technologie werden solche Matrizes aus synthetischen Polymeren, wie etwa Polyacrylat oder Polyethylen hergestellt. In diesem Zusammenhang sind auch Extrusionsverfahren zur Herstellung von Retardformen aus synthetischen Grundstoffen bekannt.

Ein prinzipieller Nachteil der bisher mittels Extrusionsverfahren hergestellten Arzneiformen liegt in der Verwendung von Wirkstoffträgern wie etwa Kunststoffen, Wachsen oder auch Fettalkoholen. Diese nicht biologisch abbaubaren und teilweise umweltschädlichen Hilfsstoffe, wie etwa Restmonomere in den verwendeten Polymeren, sind bis heute für mittels Extrusionsverfahren hergestellter Arzneiformen, die eine kontrollierte und langsame Wirkstofffreisetzung erlauben, unabdingbar. Darüber hinaus ist kein Extrusionsverfahren zur Herstellung von Arzneiformen, die eine schnelle Wirkstofffreisetzung erlauben, bekannt.

Es besteht daher ein starkes Bedürfnis, ein Extrusionsverfahren zur Herstellung von Arzneiformen mit regulierter, d.h. wahlweise verzögerter oder auch rascher Wirkstofffreisetzung zur Verfügung zu stellen, welches die Nachteile des Standes der Technik überwindet, insbesondere die Verwendung von nicht biologisch abbaubaren und teilweise sogar umwelttoxischen Trägerstoffen vermeidet. Weitere Aufgaben ergeben sich aus der nachfolgenden Beschreibung der Erfindung.

Die Lösung der verfahrensbezogenen Aufgaben liegt in den Merkmalen des Anspruchs 1.

Vorteilhafte Ausgestaltungen des erfindungsgemäßen Verfahrens sind in den Verfahrensunteransprüchen definiert.

Erfindungsgemäß wird jetzt ein Verfahren zur Herstellung von Arzneiformen oder Vorstufen davon mittels Extrusion vorgeschlagen, das dadurch gekennzeichnet ist, daß die Arzneiform eine den Wirkstoffgehalt im wesentlichen enthaltende Matrix aufweist, die in ihren wesentlichen Eigenschaften durch die Extrusion ausgebildet wird, und ein biologisch abbaubares Polysaccharid und/oder Derivat davon und/oder einen Komplex davon und/oder eine beliebige Mischung der vorgenannten Substanzen mit anderen Substanzen und/oder Sacchariden und/oder Derivaten davon als Bestandteil der Matrix und mindestens eine pharmazeutisch wirksame Substanz umfaßt.

In einer bevorzugten Ausführungsform wird die Wirkstofffreisetzung der Arzneiform durch Zusetzen von Hilfsstoffen und/oder Variieren der Extrusionsprozeßparamater wie Temperatur, Düsengeometrie und/oder Extrusionsgeschwindigkeit reguliert.

In einer weiteren bevorzugten Ausführungsform ist die Matrix der erfindungsgemäß hergestellten Arzneiform amorph oder teilamorph.

Weiter betrifft die Erfindung in einer bevorzugten Ausführungsform ein Verfahren, bei dem die erfindungsgemäß hergestellte Arzneiform als Polysaccharid Stärke oder ein Derivat davon, insbesondere amorphe oder teilamorphe Stärke oder ein Derivat davon, in der Matrix umfaßt.

In einer weiteren besonders bevorzugten Ausführungsform betrifft die Erfindung ein Herstellungsverfahren, bei dem die Arzneiform eine wasserunlösliche und vorzugsweise quellbare Matrix umfaßt.

In einer besonders bevorzugten Ausführungsform handelt es sich bei der erfindungsgemäß hergestellten Arzneiform um eine Retardmatrix.

Weitere bevorzugte Ausführungsformen liegen in einer erfindungsgemäß hergestellten Arzneiform, die eine im wesentlichen der Lapidus-Funktion gehorchende Wirkstofffreisetzung und besonders bevorzugt eine über 24 h oder länger einstellbare Wirkstofffreisetung aufweist.

Eine weitere wesentliche Aufgabe der Erfindung liegt in der Bereitstellung einer Arzneiform, die im wesentlichen ohne nicht biologisch abbaubare Inhaltsstoffe auskommt.

Diese Aufgabe wird erfindungsgemäß durch eine Arzneiform gelöst, die eine den Wirkstoffgehalt im wesentlichen enthaltende Matrix, die in ihren wesentlichen Eigenschaften durch Extrusion ausgebildet ist, und ein Polysaccharid und/oder ein Derivat davon und/oder einen Komplex davon und/oder eine beliebige Mischung der vorgenannten Substanzen mit anderen Substanzen und/oder Sacchariden und/oder Derivaten davon als wesentlichen Bestandteil der Matrix sowie mindestens eine pharmazeutisch wirksame Substanz umfaßt.

Weiter betrifft die Erfindung die Verwendung der erfindungsgemäßen Arzneiform als Hilfsstoff bei der Direkttablettierung, zur Herstellung von Granulaten für die Tablettierung und Kapselfüllung, zur Weiterverarbeitung mittels Spritzgußverfahren und/oder zur Herstellung von Monoblockarzneiformen.

Während des Extrusionsverfahrens, also unter Anwendung bzw. Ausübung von Wärme, von Scherkräften und Druck wird eine amorphe oder teilamorphe Matrix aus den kristallinen oder teilkristallinen Polysacchariden, insbesondere aus Stärke oder Derivaten davon, bzw. aus Mischungen dieser Komponenten generiert. Den Extrusionsbedingungen wie Temperatur, Düsengeometrie und Extrusionsgeschwindigkeit kommen bei der reproduzierbaren Herstellung von Arzneiformen, die auf der Extrusion von Polysacchariden und Derivaten davon basieren, eine wichtige Bedeutung zu. Beispielsweise kann native Stärke bei geeigneten Extrusionsbedingungen komplett plastifiziert oder verglast werden, so daß homogene kunststoffartige Formkörper entstehen.

Bei der Extrusion von Stärke ist eine Heizung nur zu Beginn des Prozesses notwendig. Im weiteren Verlauf des Prozesses wird vorzugsweise die durch die starke Scherung und Friktion entstehende Wärme durch Kühlung abgeführt, um die Temperatur konstant zu halten. Die bei dem erfindungsgemäßen Verfahren eingesetzten Formulierungen bestehen aus einer Mischung von Polysacchariden und/oder Derivaten davon, bevorzugt aus einer Mischung von Stärke und/oder Stärkederivaten, wobei verschiedenste Stärketypen geeignet sind. Des weiteren beinhaltet die Mischung mindestens eine pharmazeutisch wirksame Substanz in Mengen von bis zu 50 % und bevorzugt von bis zu 30 % bezogen auf das Gesamtgewicht der Formulierung, und kann außerdem noch weitere andere Substanzen umfassen. Der gründlich durchgemischten Trockenformulierung sollte Wasser in Konzentrationen von bis zu 15 % zugesetzt werden. Bei Verwendung eines zwangsfördernden Extruders ist ein geringerer Wasseranteil als 15 % ausreichend.

Nach der gründlichen Durchmischung und dem Zusatz von Wasser sollte die erhaltene Vormischung vorzugsweise klumpenfrei gesiebt werden, um eine einwandfreie Förderung durch die Stopfschnecke sicherzustellen. Das Anfahren und Reinigen des Extruders kann bspw. mit Maisgrieß erfolgen. Bei der Generierung einer Matrix nach dem erfindungsgemäßen Verfahren sollte die Temperatur am Austritt des Extruders unter Normaldruck 100° C nicht überschreiten, da bei Temperaturen oberhalb von 100° C die Bildung einer porenfreien Matrix nur sehr schwer möglich sein dürfte. Die dem Prozeß insgesamt zuzuführende Gesamtenergie, in Form von Scherkräften, Temperatur, Wärme oder Druck sollte möglichst konstant und zum Erreichen des Glasüberganges ausreichend sein. Die jeweils optimale Schneckendrehzahl und Düsengeometrie kann auf die Mischung aus erfindungsgemäßem Träger und Wasser abgestimmt werden. Bei Extrusionstemperaturen unterhalb von 100° C und geeigneter Schneckendrehzahl erhält man meist durchsichtige und vollkommen amorphe Produkte. Der Grad der Plastifizierung der Wirkstoff/Polysaccharid-, bevorzugt Wirkstoff/Stärke-, Mischungen ist eng mit der Schneckendrehzahl verknüpft. Während unterhalb der kleinsten Drehzahl eine Extrusion nicht mehr möglich ist, führt eine zu hohe Schneckendrehzahl zum teilweisen "Aufpoppen" der Arzneiform.

Das Variieren der Extrusionsprozeßparameter ermöglicht die Herstellung von Arzneiformen nach dem erfindungsgemäßen Verfahren, die eine regulierte Wirkstofffreisetzung aufweisen. Im Sinne der vorliegenden Erfindung bedeutet "reguliert", daß durch das erfindungsgemäße Extrusionsverfahren sowohl rasch freisetzende Arzneiformen als auch verzögert freisetzende Arzneiformen, sog. Retard-Arzneiformen mit Freisetzungszeiten von bis zu 24 Stunden oder länger hergestellt werden können. Neben den bereits genannten Prozeßparametern kommt auch der Verarbeitungstemperatur im Bezug auf die Regulierung der Wirkstofffreisetzung eine wichtige Bedeutung zu. Schnell freisetzende Arzneiformen erhält man z.B. durch Extrusion unterhalb der Verkleisterungstemperatur der jeweils verwendeten Polysaccharide. Da u.a. die Dichte der Matrix die Freisetzung der inkorporierten Wirkstoffe beeinflußt, können entsprechende Retard-Arzneiformen durch teilweise bis vollständige Verglasung, d.h. durch Übergang in den amorphen Zustand, des Polysaccharid-enthaltenden Gemisches bei geeigneten Extrusionsbedingungen hergestellt werden. Im Gegensatz zu bisher beschriebenen Systemen ist eine solche nach dem erfindungsgemäßen Verfahren hergestellte, amorphe oder teilamorphe Matrix, bevorzugt aus amorpher oder teilamorpher Stärke oder Derivaten davon bzw. aus Mischungen dieser Komponenten, im wesentlichen nicht wasserlöslich, sondern vorzugsweise quellbar und wasserunlöslich. Die pharmazeutisch wirksame Substanz bzw. die pharmazeutisch wirksamen Substanzen können in der Matrix in gelöster, fester oder flüssiger Form vorliegen.

Beispiele für Stärken, die im Rahmen des erfindungsgemäßen Herstellungsverfahrens eingesetzt werden können, sind Tapiokastärke, Weizenstärke, Kartoffelstärke, Starch 1500® (partiell vorverkleisterte/pregelatinisierte Maisstärke vertrieben von der Firma Colorcon), Waxylis® (Wachsmaisstärke der Firma Roquette), Eurylon 7® (Amylomaisstärke der Firma Roquette), Maisstärke, Acetylstärke.

Nach dem erfindungsgemäßen Extrusionsverfahren sind außerdem Arzneiformen erhältlich, die eine durch das Zusetzen von Hilfsstoffen regulierte Wirkstofffreisetzung aufweisen. Zur Beschleunigung der Wirkstofffreisetzung durch Porenbildung in der Matrix können beispielsweise zugesetzt werden:
- hydrophile oder amphiphile Feststoffe, wasserlösliche Stoffe wie z.B. Natriumchlorid, Lactose, oberflächenaktive Stoffe wie z.B. Natriumlaurylsulfat, Siliziumdioxid (kolloiddispers und/oder als Xerogel),
- hydrophile oder amphiphile Flüssigkeiten, Polyglykole wie z.B. Glycerol, Polyethylenglykol, oberflächenaktive Stoffe wie z.B. Polysorbate,
- Gase wie z.B. Stickstoff, Kohlendioxid.

Eine Verminderung der Wirkstofffreisetzung durch Behinderung der Diffusion kann beispielsweise durch Zusetzen folgender Hilfstoffe bewirkt werden:
- lipophile oder amphiphile Hilfsstoffe natürlichen, synthetischen und/oder partialsynthetischen Ursprungs wie z.B. Fette, Fettsäuren, Wachse in festem oder flüssigen Zustand,
- gesättigte oder ungesättigte Kohlenwasserstoffe,
- Metallseifen wie z.B. Magnesiumstearat.

Des weiteren kann eine Verminderung der Wirkstofffreisetzung durch Ausbildung stöchiometrischer oder unstöchiometrischer Komplexe wie z.B. Jod-Stärke-Komplex, Miltefosin-Amylose-Komplex erreicht werden.

Darüberhinaus kann die Freisetzung der inkorporierten Wirkstoffe durch Einsatz geeigneter Mischungen der Bestandteile der Matrix, bevorzugt durch Verwenden von Mischungen verschiedener Stärken und/oder Derivaten davon, beeinflußt werden. Weitere Faktoren, die die Wirkstofffreisetzung zusätzlich beeinflussen können, sind die Oberfläche des Formkörpers der Arzneiform und deren Partikelgröße bzw. die Verteilung des Wirkstoffes bzw. der Wirkstoffe innerhalb des Partikels.

Die Überprüfung des Verlaufs der Wirkstofffreisetzung zeigt, daß die Freisetzung des Wirkstoffs bevorzugt diffusionsgesteuert ist und der sogenannten Lapidusregel gehorcht, wie die nachfolgend beschriebenen Versuche zeigen. Der Freisetzungsverlauf ändert sich auch nach mehrmonatiger Lagerung der Arzneiformen nicht.

Die nach dem erfindungsgemäßen Verfahren hergestellten Arzneiformen können zur Herstellung von Granulaten für die Tablettierung und Kapselfüllung, als Hilfsstoffe zur Direkttablettierung, zur Weiterverarbeitung mittels Spritzgußverfahren und/oder als Monoblockarzneiformen, bei denen die Ausformung des Extrudats durch eine geeignete Anordnung an der Extruderdüse - ähnlich wie bei der Seifenproduktion - erfolgt, verwendet werden. Das erfindungsgemäße Verfahren ermöglicht die Herstellung und Verwendung von Monoblockformen. Dabei entsteht ein Extrudat, das nur an der Oberfläche verglast ist und im Inneren die Wirkstoff-Träger-Mischung in unverändertem Zustand einschließt. Zur Erzielung der jeweils gewünschten, biopharmazeutischen Eigenschaften der Arzneiform können alle für die Herstellung von festen Formen bekannten Hilfsstoffe verwendet werden.

Die Trocknung der Arzneiform kann allein über die entstehende Friktionswärme erfolgen, so daß kein finaler Trocknungsschritt notwendig ist. In einem Produktionsextruder, bevorzugt in einem Doppelschneckenextruder, besonders bevorzugt in einem zwangsfördernden Doppelschneckenextruder, können alle Prozeßschritte, wie Dosieren, Befeuchten, Mischen, Extrudieren und Formen kontinuierlich ablaufen. Das erfindungsgemäße Verfahren kann somit den Misch-, Granulier- und Trocknungsprozeß, für den keine zusätzliche Energie aufgewendet werden muß, in einem Gerät vereinigen.

Erfindungsgemäß wird auch die Zahl möglicher Inkompatibilitäten reduziert, da die Arzneiform beispielsweise nur einen Trägerstoff, bevorzugt Stärke oder ein Derivat davon, und eine pharmazeutisch wirksame Substanz umfaßt. Die nach dem erfindungsgemäßen Verfahren hergestellte Matrix verhindert außerdem eine mögliche, ungewollte Entmischung.

Die nachfolgenden Beispiele zeigen, daß der Grad der Wirkstoffretardierung über den Prozeß bzw. die Prozeßparameter, den Polysaccharidtyp oder den Zusatz weiterer Hilfsstoffe gut regulierbar ist. Die Beispiele dienen der Veranschaulichung der Erfindung und stellen keine Einschränkungen auf bestimmte Ausführungsformen bzw. Anwendungsbereiche dar. Vielmehr kann man sich weitere Ausführungsformen innerhalb des durch die beigefügten Patentansprüche definierten Schutzumfangs vorstellen.

### Beispiele

Für die folgenden Beispiele, die nach dem erfindungsgemäßen Verfahren durchgeführt wurden, wurden verschiedene Polysaccharide mit Zusatz von Koffein als Modellwirkstoff verwendet. Die Extrusionsversuche wurden mit einem Brabender-Einschneckenextruder vom Typ 811201 durchgeführt. Das Gerät besitzt drei voneinander unabhängig temperierbare Segmente, den Einlaßbereich, den Schneckenbereich und die Düse. Es wurde eine Förderschnecke verwendet, ohne Verdichtung, mit einer Schneckenlänge von 22 cm, einem Schneckendurchmesser von 19 mm, einem Kerndurchmesser von 16 mm und einer Ganghöhe von 15 mm. Es wurden Düsen mit verschiedenen Düsendurchmessern zwischen 2,5 bis 7 mm verwendet.

Die Ansatzgröße betrug bei den Versuchen zwischen 350 und 600 Gramm. Wirkstoff und Stärke wurden in einem Stephanmischer gründlich gemischt, mit 15 % Wasser angefeuchtet und diese Vormischung klumpenfrei gesiebt.

Temperaturen von ungefähr 65° C im Einlaßbereich des Extruders, von ca. 80° C im Schneckenbereich und von ca. 98° C an der Düse haben sich als eine geeignete Temperaturwahl zur Durchführung des erfindungsgemäßen Verfahrens erwiesen.

Der Nachweis des Überganges der kristallinen oder teilkristallinen Strukturen der Polysaccharide in amorphe oder teilamorphe Strukturen kann mit Hilfe verschiedener Verfahren nachgewiesen werden.

Mittels Differential-Thermoanalyse (DSC) kann der Übergang der kristallinen oder teilkristallinen Stärke in den amorphen Zustand detektiert werden. Bei geeigneten Extrusionsbedingungen wird die Stärke vollständig verglast, d.h. sie geht in den amorphen Zustand über. Exemplarisch wurde eine Untersuchung mit Tapiokastärke als Polysaccharid und Koffein als Wirkstoff durchgeführt. Das in Abbildung 1a gezeigte Thermogramm der Vormischung, bestehend aus 90 % Tapiokastärke und 10 % Koffein, mit Wasserzusatz weist noch die typischen endothermen Peaks im Bereich von etwa 65° C auf.

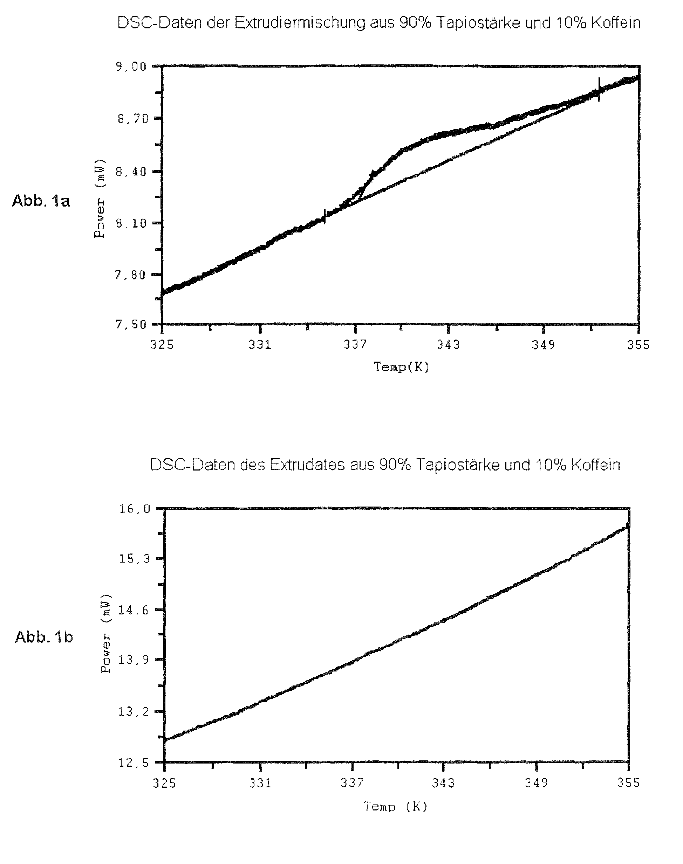

Nach der erfindungsgemäß durchgeführten Extrusion lassen sich keine Peaks im entscheidenden Temperaturbereich feststellen. Daraus ist zu folgern, daß die Stärke vollständig verglast wurde, also in den amorphen Zustand übergegangen ist (siehe Abbildung 1b).

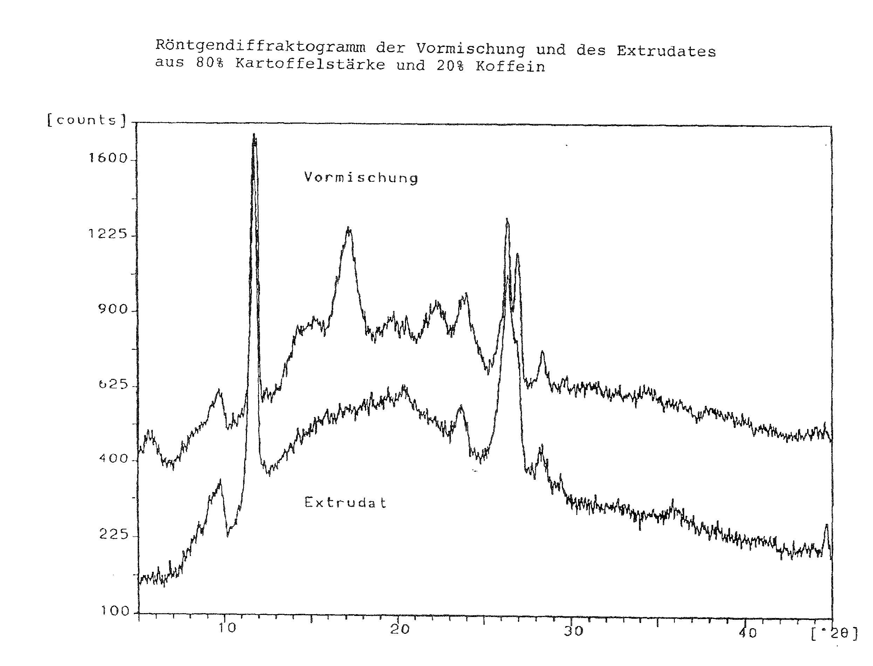

Der Übergang vom kristallinen oder teilkristallinen in den amorphen oder teilamorphen Zustand läßt sich auch mit Hilfe der Röntgendiffraktometrie nachweisen. Für die folgende exemplarische Untersuchung wurde eine Probe, bestehend aus 80 % Kartoffelstärke und
20 % Koffein verwendet. Das in Abbildung 2 dargestellte Röntgendiffraktogramm der Kartoffelstärke-Koffein-Vormischung weist im Bereich um 20° die Signale des kristallinen Anteils der Stärke auf. Nach Anwendung des erfindungsgemäßen Verfahrens zeigt das Röntgendiffraktogramm des entsprechenden Extrudats keine Signale mehr, die auf einen kristallinen Anteil der Stärke hinweisen.

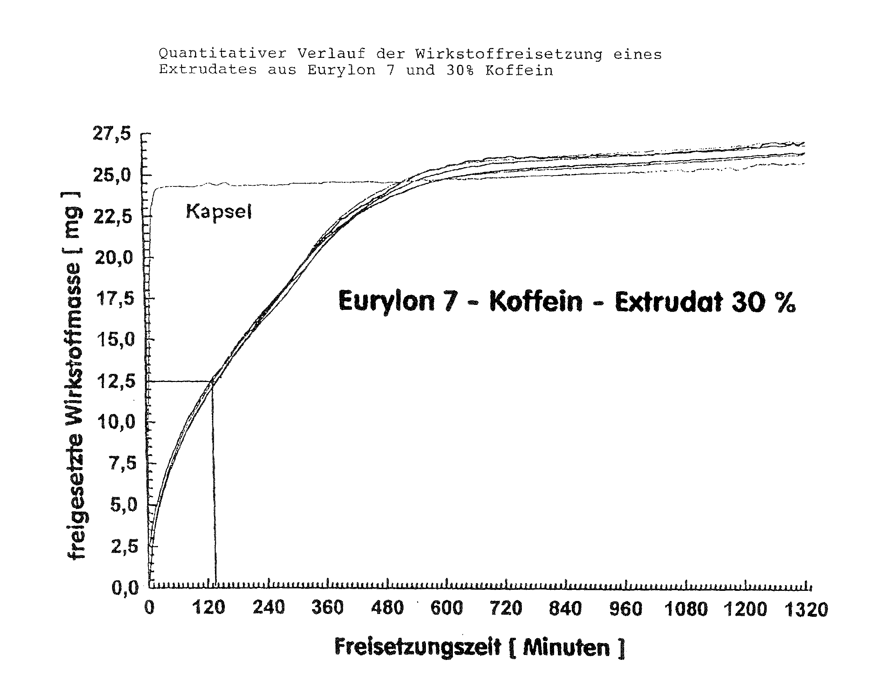

In diesem Zusammenhang sei nochmals darauf hingewiesen, daß der Grad der Destrukturierung zum Glas entscheidend für die Freisetzungskinetik der Wirkstoffe ist. Die Überprüfung der Wirkstofffreisetzung erfolgte in einem Blattrührermodell nach USP in den Flüssigkeiten 0,1 N Salzsäure (künstlicher Magensaft mit einem pH-Wert von 1) und in Phosphatpuffer pH 7,2 (künstlicher Darmsaft). In diesem Zusammenhang sei nochmals darauf hingewiesen, daß sich die Extrudate bei der Untersuchung nicht auflösten, sondern lediglich ein Aufquellen der Extrudate beobachtet wurde. Für die folgende beispielhafte Untersuchung zur Bestimmung der quantitativen Wirkstofffreisetzung wurde eine Probe, bestehend aus der kommerziell erhältlichen Stärke Eurylon 7® (Amylomaisstärke der Firma Roquette) und einem 30 %igen Anteil an Koffein verwendet. Das Diagramm in Abbildung 3 zeigt den quantitativen Verlauf der Wirkstofffreisetzung. Zum Vergleich wurde auch die Freisetzung von reinem Koffein, unretadiert abgefüllt in eine Kapsel, eingezeichnet.

Man erkennt, daß die Einbettung des Koffeins in die Polysaccharidmatrix eine erhebliche Verzögerung der Freisetzung bewirkt, in diesem Fall ausgehend von ca. 15 Minuten auf etwa 8 bis 10 Stunden für die Gesamtdosis von 25 mg.

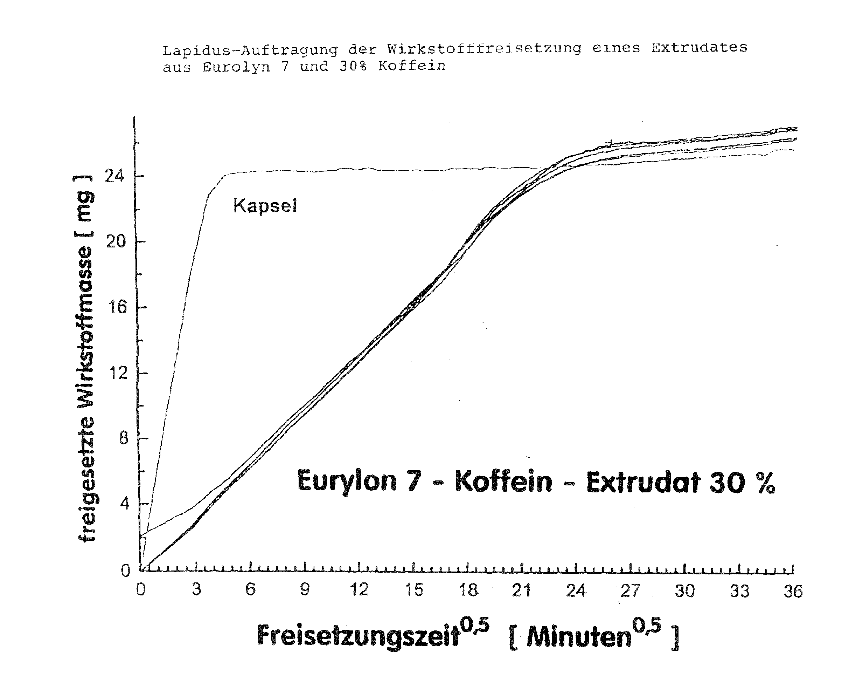

Die Auftragung der freigesetzten Menge gegen die Wurzel der Zeit entsprechend der Lapidusregel läßt einen nahezu geradlinigen Verlauf der Kurven erkennen, und es zeigt sich somit, daß die Wirkstofffreisetzung diffusionsgesteuert erfolgt (s. Abbildung 4).

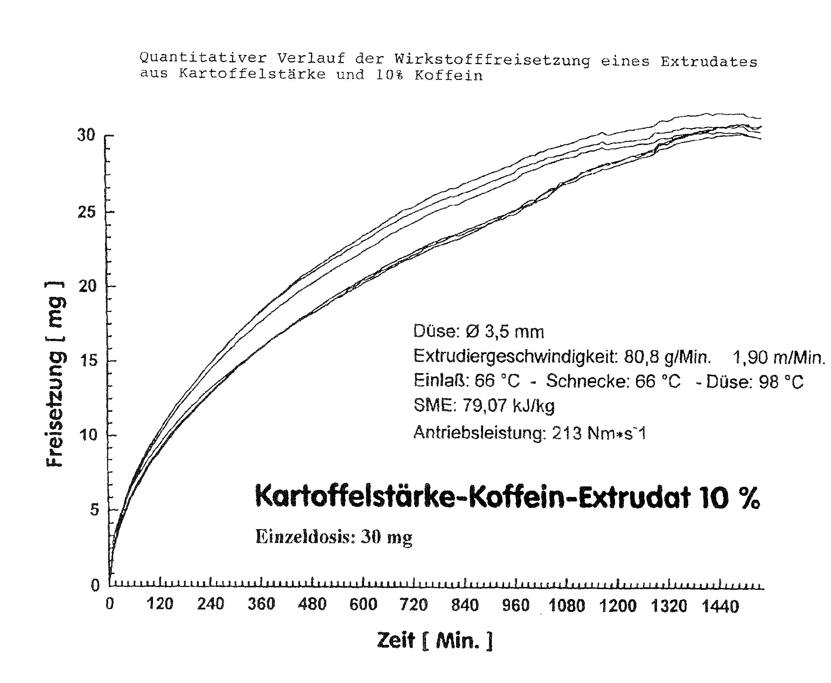

Wie bereits beschrieben, läßt sich die Geschwindigkeit der Wirkstofffreisetzung auch über die Art des Polysaccharids bzw. der Polysaccharidmischung regulieren. Im folgenden Beispiel wurde eine Vormischung, bestehend aus Kartoffelstärke und 10 % Koffein für die quantitative Untersuchung der Wirkstofffreisetzung verwendet. Der Wechsel des Stärketyps, zusammen mit der niedrigeren Wirkstoffdosierung, führt zu einer deutlichen Verlangsamung der Wirkstofffreisetzung. Das in Abbildung 5 abgebildete Diagramm zeigt, daß das nach dem erfindungsgemäßen Verfahren hergestellte Extrudat eine Wirkstofffreisetzung über einen Zeitraum von 24 Stunden bewirkt.

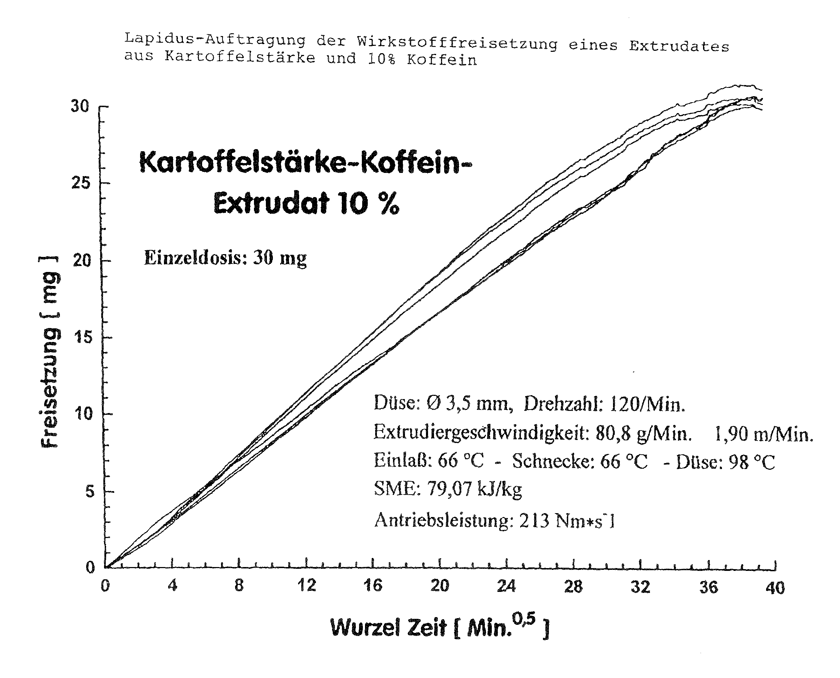

Die in Abbildung 6 dargestellte Auftragung entsprechend der Lapidusregel zeigt, daß die Wirkstofffreisetzung diffusionsgesteuert erfolgt.

Wie bereits oben beschrieben, läßt sich die Dauer und der Verlauf der Gesamtwirkstofffreisetzung regulieren. Sehr lange Wirkstofffreisetzungszeiten, wie etwa 24 Stunden bei dem vorangegangenen Beispiel, sind im Hinblick auf die Entwicklung eines neuen Retardierungsprinzips als vorteilhaft anzusehen, da man so "Reserven" für extrem gut wasserlösliche Wirkstoffe hat. Die Wirkstofffreisetzung läßt sich, wie bereits mehrfach ausgeführt, durch die verwendeten Polysaccharide bzw. entsprechende Derivaten und Mischungen davon, durch Zusatz von entsprechenden Hilfsstoffen und/oder durch die Extrusionsprozeßparameter gezielt beeinflussen.

Nach dem erfindungsgemäßen Verfahren lassen sich auch schnellere Wirkstofffreisetzungszeiten erzielen. Die unten stehende Tabelle zeigt exemplarisch, daß durch die Prozeßparameteränderung, wie beispielsweise zu einer unvollständigen Verglasung führt, die Wirkstofffreisetzungszeiten über einen weiten Bereich reguliert werden können.

**Tabelle**

| Polysaccharid | Wirkstoff | Wirkstoffkonzentration | Zeitraum in dem 50% der Wirkstoffmenge freigesetzt wird [min] | Zeitraum in dem Wirkstoff theoretisch freigesetzt wird [h] |
|---|---|---|---|---|
| Tapiokastärke | Koffein | 10% (50 mg) | 240 | 16 |
| Tapiokastärke | Koffein | 10% (50 mg) | 120 | 8 |
| Maisstärke | Koffein | 30% (50 mg) | 195 | 13 |
| Maisstärke | Koffein | 30% (50 mg) | 55 | 3.7 |

Wie aus dieser Tabelle ersichtlich, lassen sich für gleiche Stärketypen sowohl relativ kurze Wirkstofffreisetzungszeiten, als auch Wirkstofffreisetzungszeiten, die in den Bereich von Retard-Arzneiformen fallen, allein durch Variieren der Prozeßparameter erzielen.

## Patentansprüche

1. Verfahren zur Herstellung von Arzneiformen oder Vorstufen davon mittels Extrusion,
**dadurch gekennzeichnet, dass** die Arzneiform eine den Wirkstoffgehalt im wesentlichen enthaltende Matrix aufweist, die in ihren wesentlichen Eigenschaften durch die Extrusion ausgebildet wird, und ein Polysaccharid und/oder ein Derivat davon und/oder einen Komplex davon und/oder eine beliebige Mischung der vorgenannten Substanzen mit anderen Substanzen und/oder Sacchariden und/oder Derivaten davon als wesentlichen Bestandteil der Matrix sowie mindestens einen pharmazeutischen Wirkstoff umfasst, wobei die Wirkstofffreisetzung aus der Arzneiform durch Zusetzen von Hilfsstoffen und/oder Variieren der Extrusionsprozessparameter wie Temperatur, Düsengeometrie und/oder Extrusionsgeschwindigkeit reguliert wird.

2. Verfahren nach Anspruch 1,
**dadurch gekennzeichnet, dass** das Polysaccharid Stärke oder ein Derivat davon ist.

3. Verfahren nach einem der vorangehenden Ansprüche,
**dadurch gekennzeichnet, dass** die Matrix wasserunlöslich ist.

4. Verfahren nach einem der vorangehenden Ansprüche,
**dadurch gekennzeichnet, dass** die Matrix eine Retardmatrix ist.

5. Verfahren nach einem der vorangehenden Ansprüche,
**dadurch gekennzeichnet, dass** die Wirkstofffreisetzung der Arzneiform im wesentlichen gemäß der Lapidus-Funktion erfolgt.

6. Verfahren nach einem der vorangehenden Ansprüche,
**dadurch gekennzeichnet, dass** die Wirkstofffreisetzung der Arzneiform über bis zu 24 h oder länger einstellbar ist.

7. Verfahren nach einem der vorangehenden Ansprüche,
**dadurch gekennzeichnet, dass** mindestens ein pharmazeutischer Wirkstoff in gelöster, fester oder flüssiger Form in der Matrix vorliegt.

8. Arzneiform, umfassend eine den Wirkstoffgehalt im wesentlichen enthaltende Matrix, die in ihren wesentlichen Eigenschaften durch Extrusion ausgebildet ist, und ein Polysaccharid und/oder ein Derivat davon und/oder einen Komplex davon und/oder eine beliebige Mischung der vorgenannten Substanzen mit anderen Substanzen und/oder Sacchariden und/oder Derivaten davon als wesentlichen Bestandteil der Matrix sowie mindestens einen pharmazeutischen Wirkstoff, wobei die Wirkstofffreisetzung durch Zusatz von Hilfsstoffen und/oder Variationen der Extrusionsprozessparameter wie Temperatur, Düsengeometrie und/oder Extrusionsgeschwindigkeit reguliert ist.

9. Arzneiform nach Anspruch 8,
**dadurch gekennzeichnet, dass** das Polysaccharid Stärke oder ein Derivat davon ist.

10. Arzneiform nach einem der Ansprüche 8 bis 9,
**dadurch gekennzeichnet, dass** die Matrix wasserunlöslich ist.

11. Arzneiform nach einem der Ansprüche 8 bis 10,
**dadurch gekennzeichnet, dass** die Matrix eine Retardmatrix ist.

12. Arzneiform nach einem der Ansprüche 8 bis 11,
**dadurch gekennzeichnet, dass** die Wirkstofffreisetzung im wesentlichen gemäß der Lapidus-Funktion erfolgt.

13. Arzneiform nach einem der Ansprüche 8 bis 12,
**dadurch gekennzeichnet, dass** die Wirkstofffreisetzung über bis zu 24 h oder länger eingestellt ist.

14. Arzneiform nach einem der Ansprüche 8 bis 13,
**dadurch gekennzeichnet, dass** mindestens ein pharmazeutischer Wirkstoff in gelöster, fester oder flüssiger Form in der Matrix vorliegt.

15. Verwendung der Arzneiform nach Anspruch 8 bis 14 zur Herstellung von Granulaten für die Tablettierung und Kapselfüllung, zur Weiterverarbeitung mittels Spritzgussverfahren, als Hilfsstoff zur Direkttablettierung und/oder zur Herstellung von Monoblockarzneiformen.
